# EUROPEAN PATENT APPLICATION

(11) **EP 2 698 199 A1**
(43) Date of publication of application: **19.02.2014**
(21) Application number: 12005875.5
(22) Date of filing: 14.08.2012
(51) Int. Cl.: B01J 29/42, B01J 37/10, C07C 2/12

(54) **Process for dimerization of olefins**

(71) Applicant: Saudi Basic Industries Corporation, Riyadh 11422 (SA)
(72) Inventor: Jana, Suman Kumar, Manjusar 391775 District Baroda Gujarat (IN); Barochia, Jayen, Manjusar 391775 District Baroda Gujarat (IN)
(74) Representative: Modiano, Micaela Nadia

(57) **Abstract**

The present invention relates to a process for the dimerization of olefins or a mixture of olefins and paraffins, wherein said olefins and paraffins have between 2 to 10 carbon atoms, comprising contacting a feedstream comprising said olefin or mixture of olefins and paraffins with a catalyst composition comprising a medium pore aluminosilicate zeolite and up to 5 wt-% of one or more elements selected from Groups 6 and 9 of the Periodic Table.

## Description

The present invention relates to a process for the dimerization of olefins or a mixture of olefins and paraffins using a catalyst composition.

A variety of octane boosters is on the market. Currently, the most popular octane booster is methyl tertiary butyl ether (MTBE). However, because of its hazardous nature, it is predicted that the worldwide consumption of MTBE will gradually decrease. A promising candidate for replacing MTBE is isooctane. Isooctane has similar properties to MTBE, exhibits high octane number and also has very good environmental properties.

Isooctane is typically produced by dimerization of isobutene followed by hydrogenation. A few technologies are available for commercialization for the selective production of isooctenes from isobutene based on solid macro-porous acidic resin or phosphoric acid catalyst. These technologies are based on solid macro-porous acidic resin or phosphoric acid catalyst and require additive(s) and/or hydrating agent(s) together with the catalyst to obtain isooctenes selectively. However, the presence of additive(s)/hydrating agent(s) in the feed stream contributes to the formation of some amount of unwanted product in the product stream.

Catalysis Today, 100 (2005) 463 discloses catalyst H-ZSM-5 (having Si/Al ratios of 15 and 25) for isobutene dimerization under liquid-phase conditions (40°C, 10 bar pressure and n-butane as diluent). Although the catalyst shows high initial conversion, it deactivates very rapidly. The initial conversion of ∼65% drops down to < 2% within 1 h of reaction, hence it is not suitable for industrial scale dimerization of isobutene.

EP 1167326 A1 discloses the dimerization of isobutene using a catalyst system comprising a zeolite with TON-type structure. The catalyst was unable to show the desired product selectivity for a long time. The dimer selectivity decreased from 72% to 54% within 4 h of reaction. The catalyst also got deactivated fast. Isobutene conversion decreased from 98% to 84% within 4 h of reaction.

WO 2004/080935 A1 discloses medium pore zeolites. H-ZSM-5, H-ZSM-22, H-ZSM-23, Ferrierite having Al content ∼ 0.1 to 5 wt% are reported for isobutene dimerization under high pressure reaction conditions. However, these catalysts deactivate fast and hence require frequent regeneration for long reaction process.

WO09027582 discloses a process for oligomerizing olefinic, lower hydrocarbons using an acidic catalyst selected from the group of natural and synthetic zeolites or from the mesoporous aluminosilicates. Zeolite is selected from the group consisting of ZSM-5, ZSM-22, ZSM-23, ferrierite and ion-exchanged zeolites prepared therefrom.

WO200480935 discloses a process for dimerizing lower, olefinic hydrocarbons with a medium pore zeolite under process conditions allowing selective dimerization. The olefinic hydrocarbon feedstock is contacted with an acid catalyst at conditions in which at least a part of the olefins dimerizes. The effluent from the reaction zone is conducted to the separation zone where dimerized reaction product is separated from said effluent.

US3325465 discloses a process for dimerizing isobutene using 13X molecular sieve wherein the 95.9% of sodium ion is counter ion exchanged with cobalt ion.

The major disadvantages of the known prior art are low isooctene selectivity and low catalyst stability. Also the use of ion-exchange resin catalysts requires cumbersome catalyst regeneration, which leads to the generation of more effluents.

It is an object of the present invention to provide an improved process for the dimerization of olefins or a mixture of olefins and paraffins.

Accordingly, the present invention provides a process for the dimerization of olefins or a mixture of olefins and paraffins, wherein said olefins and paraffins have between 2 to 10 carbon atoms, comprising contacting a feedstream comprising said olefin or mixture of olefins and paraffins with a catalyst composition comprising a medium pore aluminosilicate zeolite and up to 5 wt-% of one or more elements selected from Groups 6 and 9 of the Periodic Table.

According to the process of the present invention, a high selectivity for the dimerization (e.g. from isobutene to isooctene (2,4,4-Trimethyl pentene)) is obtained. It is also advantageous that the feed stream does not require the presence of additive(s)/hydrating agent(s) which leads to the production of unwanted product(s).

### Zeolite

The zeolite used in the present invention is crystalline materials. Crystalline materials are preferred because of their regular pore size and channelling framework structures.

As used herein, the term "zeolite" or "aluminosilicate zeolite" relates to an aluminosilicate molecular sieve. These inorganic porous materials are well known to the skilled person. An overview of their characteristics is for example provided by the chapter on Molecular Sieves in Kirk-Othmer Encyclopedia of Chemical Technology, Volume 16, p 811-853; in Atlas of Zeolite Framework Types, 5th edition, (Elsevier, 2001).

Aluminosilicate zeolites are generally characterized by the Si/Al ratio of the framework. This ratio may vary widely in the catalyst composition used in the method according to the invention. Preferably, the silicon to aluminium (Si:Al) molar ratio of the zeolite is from about 5 to 1000, more preferably from about 8 to 500.

More preferably, the Si:Al molar ratio of the zeolite is 10-100, preferably 30-60 and most preferably 40-50. This molar ratio of the zeolite is very useful as the zeolite with Si/Al ratio in this range contains moderate acidity and assists in promoting acid catalyzed isobutene dimerization process selectively. This is achieved mostly by preventing unwanted oligomerization reaction through suppressing the severity of the reaction.

Any aluminosilicate that shows activity in the dimerization of olefins may be applied. Examples of suitable materials include the mordenite framework inverted (MFI) and other zeolite structures known to the skilled person, for example MEL, MWW, BEA, MOR, LTL and MTT type. Preferred materials are those known as ZSM-5, ZSM-11, ZSM-8, ZSM-12, ZSM-22, ZSM-23, ZSM-35, ZSM-38, and beta. Most preferably the zeolite is a MFI type zeolite, for example a ZSM-5 zeolite.

The term "medium pore zeolite" is commonly used in the field of zeolite catalyst compositions. A medium pore size zeolite is a zeolite having a pore size of 5-6 Å. Preferably, the zeolite has a pore size of 5-6 Å, more preferably a pore size of 5.2-5.8 Å. Medium pore size zeolites are 10-ring zeolites. i.e. the pore is formed by a ring consisting of 10 SiO₄ tetrahedra. Zeolites of the 8-ring structure type are called small pore size zeolites; and those of the 12-ring structure type, like for example beta zeolite, are referred to as large pore sized. In the above cited Altlas of Zeolite Framework Types, various zeolites are listed based on ring structure. Preferably, the zeolite is a medium pore size aluminosilicate zeolite.

The zeolite used in the present invention may be dealuminated. Means and methods to obtain dealuminated zeolite are well known in the art and include, but are not limited to the acid leaching technique; see e.g. Post-synthesis Modification I; Molecular Sieves, Volume 3; Eds. H. G. Karge, J. Weitkamp; Year (2002); Pages 204-255. Preferably, the zeolite is a dealuminated zeolite having a SiO₂ to Al₂O₃ molar ratio of 10 to 200, for improving the performance/stability of the catalyst composition. Means and methods for quantifying the SiO₂ to Al₂O₃ molar ratio of a dealuminated zeolite are well known in the art and include, but are not limited to AAS (Atomic Absorption Spectrometer) or ICP (Inductively Coupled Plasma Spectrometry) analysis.

The zeolite used in the present invention is in the hydrogen form: i.e. having at least a portion of the original cations associated therewith replaced by hydrogen. Methods to convert an aluminosilicate zeolite to the hydrogen form are well known in the art. One method involves base-exchange using ammonium salts followed by calcination.

The zeolite used in the present invention comprises up to 5 wt-% of one or more elements selected from Groups 6 and 9 of the Periodic Table, i.e. chromium, molybdenum, tungsten, seaborgium, cobalt, rhodium, iridium and meitnerium. This results in a high selectivity towards isooctene. Preferably, said one or more elements are selected from the group consisting of molybdenum, tungsten, cobalt and rhodium.

The amount of these elements in the zeolite may e.g. be at most 5 wt-%, 4 wt-%, 3-wt%, 2-wt%, 1.5 wt%, 1.25 wt%, 1.1 wt% or 1.0 wt%. The amount of these elements in the zeolite may e.g. be at least 0.05 wt%, 0.1 wt%, 0.25 wt% or 0.5 wt%.

These elements may be present in the zeolite structure as framework or non-framework element; as counter ion in the zeolite; on its surface, e.g. in the form of metal oxides; or be present in a combination of these forms.

Preferably, the catalyst used in the present invention has the framework structure of ZSM-5 with Bronsted acid sites provided by tetrahedrally coordinated aluminium in the framework structure and Lewis and/or Bronsted acid sites provided by elements from Groups 6 and 9 of the Periodic table in the framework and/or non-framework structure.

The zeolite catalyst used in the present invention can be prepared by suitable methods of preparing and modifying zeolites as well known to the skilled person; including for example impregnation, calcination, steam and/or other thermal treatment steps. Such methods are disclosed for instance in documents US 7186872B2; US4822939 and US4180689 hereby incorporated by reference. The zeolite catalyst used in the present process can also be made by ion exchange technique, sonification technique, precipitation technique, which are all well known to the skilled person.

The catalyst composition comprises a zeolite catalyst as described above. The catalyst composition may consist of the zeolite catalyst as described above, or the catalyst composition may comprise further components such as diluents or binders or other support materials. Preferably these further components do not negatively affect the catalytic performance of the catalyst composition of the invention. Such components are known to the skilled person.

For example, the catalyst composition of the invention may further comprise a non- acidic inert diluent. Preferably the non-acidic inert diluent is silica.

Examples of suitable binder materials include metal oxides, mixed metal oxides, clays, metal carbides and metal oxide hydroxides. The metal oxide or the mixed metal oxides may be chosen from the group of metal oxides comprising for example, oxides of magnesium, aluminium, titanium, zirconium and silicon. The clay may be, but is not limited to, kaolin, montmorillonite or betonite. Metal carbides suitable for use in the composition of the invention are, for example, molybdenum carbide and silicon carbide. The metal oxide hydroxide may be feroxyhyte or Goethite, or more preferably boehmite.

The binder may be present in the composition according to the invention in for example at least 5 wt %, for example at least 10 wt%, for example at least 20 wt %, for example at least 30 wt %, for example at least 40 wt %, for example at least 50% and/or for example at most 5 wt %, for example at most 10 wt%, for example at most 20 wt %, for example at most 30 wt %, for example at most 40 wt %, for example at most 50 wt% with respect to the total catalyst composition.

If the zeolite catalyst composition is to contain a binder, such catalyst composition can be obtained, for example, by mixing the zeolite and a binder in a liquid, and forming the mixture into shapes, like pellets or tablets, applying methods known to the skilled person.

### Pre-treatment step

In the process according to the present invention, the catalyst composition may be pre-treated before being contacted with the feedstream. The catalyst composition may be, before contacting with the feedstream, pre-treated with an inert gas comprising water vapour or alcohol vapour at a temperature between 30°C and the boiling temperature of water or the alcohol.

According to the pre-treatment of this embodiment, the relatively strong acid sites of zeolite catalyst is reduced through control adsorption of water or alcohol molecule on the acidic sites. This results in that the severity of the dimerization reaction is minimized. The pre-treatment of the catalyst with an inert gas comprising water or alcohol vapour hence leads to a catalyst with a higher selectivity towards dimers, e.g. contacting isobutene with the pre-treated catalyst composition will result in a high selectivity towards isooctene (2,4,4-Trimethyl pentene). It is also advantageous that the feed stream does not require the presence of additive(s)/hydrating agent(s) which leads to the production of unwanted product(s).

The contact temperature of the zeolite with the inert gas comprising water vapour or alcohol vapour is preferably 30-90 °C, more preferably 40-70 °C.

The inert gas is preferably saturated with water vapour or alcohol vapour.

The alcohol is preferably selected from the group consisting of 1-propanol, isopropanol, isobutanol and tertiary butanol or a mixture thereof.

The inert gas is selected from the group consisting of nitrogen, helium and argon or a mixture thereof.

### Optional contacting with dry gas

The pre-treatment may further comprise the step of contacting the zeolite with an inert gas which does not comprise water vapour or alcohol vapour (hereinafter sometimes referred as "dry inert gas" or "dry gas") before and/or after the contacting step with the inert gas comprising water or alcohol vapour. The contacting step with the dry inert gas may be performed at the same as or different from the temperature of the contacting step with the inert gas comprising water vapour or alcohol. For example, the contacting step with the dry gas may be performed at 30 to 350 °C. The duration of the contacting step may e.g. be 0.1 to 3 hours.

### Contacting with dry gas before contacting with the moist gas

The contacting step with the dry inert gas before the contacting step with the inert gas comprising water or alcohol vapour is preferably performed at a temperature between 100 to 300 °C. Contacting the catalyst composition at a temperature between 100 to 300 °C before contacting with the inert gas comprising water or alcohol vapour helps to quick removal of pre-adsorbed gases from the catalyst, resulting in cleaning the catalyst surface.

The contacting step with the dry inert gas before the contacting step with the inert gas comprising water or alcohol vapour may e.g. be 0.3 to 1.8 hours. It was found that this range is suitable to remove pre-adsorbed gases from the catalyst.

### Contacting with the moist gas

The contacting step with the inert gas comprising water or alcohol vapour is performed at a temperature between 30 °C to the boiling temperature of water/alcohol, preferably 30-90 °C, more preferably 40-70 °C.

The contacting step with the inert gas comprising water or alcohol vapour is preferably performed for 0.2 to 2.0 hours. It was found that this range results in a combination of good isobutene conversion rate and good selectivity. Particularly preferred is contacting for 0.4 to 1.0 hours, which results in a combination of good conversion rate and very high selectivity towards dimer.

### Contacting with dry gas after contacting with the moist gas

The contacting step with the dry inert gas after the contacting step with the inert gas comprising water or alcohol vapour is preferably performed at a temperature between 50 °C and the boiling temperature of water or the alcohol. Particularly preferred is the contacting at the boiling temperature of water or the alcohol or at a temperature at most 10 °C lower than the boiling temperature of water or the alcohol, which results in a combination of good conversion and very high selectivity towards dimer.

The contacting step with the dry inert gas after the contacting step with the inert gas comprising water or alcohol vapour may e.g. be 0.1 to 1.5 hours. Particularly preferred is contacting for 0.5 to 1.0 hours, which results in a combination of good conversion and very high selectivity towards dimer.

Particularly preferred embodiments include a pre-treatment method comprising contacting with the dry gas at 300 °C for 1 h, subsequently contacting with the moist gas at 50 °C for 1 h, and subsequently contacting with the dry gas at the boiling temperature of water or the alcohol for 0.5 h.

A further aspect of the present invention provides the pre-treated catalyst composition as used in the process according to the present invention. Accordingly, the present invention provides a pre-treated catalyst composition comprising a medium pore aluminosilicate zeolite and up to 5 wt-% of one or more elements selected from Groups 6 and 9 of the Periodic Table, wherein the catalyst composition is pre-treated with an inert gas comprising water vapour or alcohol vapour at a temperature of 30 °C to the boiling temperature of water/alcohol, preferably 30-90 °C, more preferably 40-70 °C

Preferably, the molar ratio of the olefins to paraffins in the feedstream is 1:0.1-10, more preferably 1:0.2-5, and most preferably 1:0.5-2.

Preferably, the feedstream comprises isobutene and isobutane.

The feed stream may further contain one or more diluents, the concentration of which may vary over wide ranges; preferably the feed stream comprises 10-90 vol % of a feed diluent. Examples of suitable diluents include helium, nitrogen, carbon dioxide, and water.

The step of contacting the feed stream with the treated catalyst composition can be performed in any suitable reactor, as known to a skilled man, for example in a fixed bed, a fluidized bed, or any other circulating or moving bed reactor. With reactor is meant a device for containing and controlling a chemical reaction, in this case the dimerization of olefins such as isobutene.

The step of contacting the feed stream with the catalyst composition is performed at olefin dimerization conditions. These conditions are known from the prior art. A higher temperature generally enhances conversion and formation of oligomers. However, higher temperatures may induce side-reactions or promote deactivation of the catalyst, Therefore, the contacting step is preferably performed at a temperature of 40-80 °C.

Suitable pressures to conduct the contacting step are from between 1-2.5 MPa.

The flow rate at which the feed stream is fed to the reactor may vary widely, but is preferably such that a liquid hourly space velocity (LHSV) results of about 0.1 - 100 h⁻1, more preferably LHSV is about 0.5 -50 h⁻¹, or 1 - 20 h⁻¹ or most preferably 7.5-15 h⁻¹. The LHSV may be preferably at least 0.1 h⁻¹, for example at least 10 h⁻¹, for example at least 20 h⁻¹, for example at least 30 h⁻¹, and/or for example at most 1 h⁻¹, for example at most 10 h⁻¹, for example at most 20 h⁻¹, for example at most 30 h⁻¹, for example at most 40 h⁻¹, for example at most 50 h⁻¹. LHSV is the ratio of the rate at which the feed stream is fed to the reactor (in volume per hour) divided by the weight of catalyst composition in a reactor; and is thus inversely related to contact time. By contact time is meant the period of time during which the feedstream is in contact with the catalyst composition.

The LHSV indicates that there is a certain rate at which the feedstream is fed to the reactor. The total length of time in which the feedstream is fed to the reactor is known as the "Time-on-Stream (TOS)." The TOS may be for example at least 2 hours, for example at least 10 hours, for example at least 50 hours, for example at least 100 hours and/or for example at most 2 hours, for example at most 10 hours, for example at most 50 hours, for example at most 100 hours. For example the TOS for a catalyst composition according to the invention during which time the catalyst composition maintains its activity in terms of a high conversion and high selectivity for isooctene, ranges from for example 10 to 100 hours, for example from 15 to 50 hours.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention will now be further illustrated with below described experiments.

### Example 1

Various metal incorporated H-ZSM-5 (Si/Al ratio of 45, metal loading of about 0.75%) zeolites prepared by wetness impregnation technique shown in Table 1 were used for isobutene dimerization.

Pre-calcined samples as shown in Table 1 were pre- treated at 300°C for 1 h under dry N₂, followed by 50°C for 1 h under moist N₂ and subsequently at 100°C for 0.5 h under dry N₂.

A feed of isobutene diluted with isobutane (50 : 50 weight ratio) was contacted with the pre-treated catalysts at 50 °C and 20 bar for 2.5 hours.

**Table 1:**

| Zeolite catalysts | Isobutene conversion /% | Selectivity/% | | | |
|---|---|---|---|---|---|
| | | C₈ | | C₁₂ | C_{16 &+} |
| | | 2,4,4-Trimethyl pentene | Others | | |
| Co / H ZSM-5 | 43.1 | 92.2 | 0.3 | 6.6 | 0.9 |
| W / H ZSM-5 | 36.7 | 90.6 | 0 | 9.4 | 0 |
| Rh / H-ZSM-5 | 38.8 | 92.3 | 0.5 | 6.8 | 0.4 |
| H-ZSM-5 (45) | 47.0 | 88.9 | 0.6 | 8.4 | 2.1 |

### Example 2

Various metal incorporated H-ZSM-5 (Si/Al ratio of 45, metal loading of about 0.75%) zeolites prepared by ion-exchange technique shown in Table 2 were used for isobutene dimerization.

The conditions for the pre-treatment and the dimerization were the same as in Example 1.

**Table 2**

| Zeolite catalysts | Isobutene conversion /% | Selectivity / % | | | |
|---|---|---|---|---|---|
| | | C₈ | | C₁₂ | C_{16 & +} |
| | | 2,4,4-Trimethyl pentene | Others | | |
| Co / H-ZSM-5 | 54.4 | 91.2 | 0.5 | 7.9 | 0.4 |
| W / H ZSM-5 | 52.4 | 88.1 | 0.5 | 11.4 | 0 |
| Zr/H-ZSM-5 | 27.2 | 95.2 | 0 | 4.8 | 0 |
| Rh / H ZSM-5 | 52.4 | 90.5 | 0.4 | 7.4 | 1.7 |
| Mo /H-ZSM-5 | 50.4 | 92.4 | 0.4 | 7.1 | 0.1 |

### Example 3

H-ZSM-5 (Si/Al ratio of 45) zeolites comprising various amounts of Co prepared by ion-exchange technique as shown in Table 3 were used for isobutene dimerization.

The conditions for the pre-treatment and the dimerization were the same as in Example 1.

**Table 3**

| Amount of Co loading / % | Isobutene conversion / % | Selectivity / % | | | |
|---|---|---|---|---|---|
| | | C₈ | | C₁₂ | C_{16 & +} |
| | | 2,4,4-Trimethyl pentene | Others | | |
| 0.5 | 50.9 | 89.3 | 0.4 | 10.2 | 0.1 |
| 0.75 | 54.4 | 91.2 | 0.5 | 7.9 | 0.4 |
| 1.0 | 51.5 | 92.0 | 0.2 | 5.5 | 2.3 |
| 1.25 | 48.1 | 92.1 | 0.3 | 7.6 | 0 |

### Example 4

H-ZSM-5 (Si/Al ratio of 45) zeolites comprising various amounts of W prepared by ion-exchange technique as shown in Table 4 were used for isobutene dimerization.

The conditions for the pre-treatment and the dimerization were the same as in Example 1.

**Table 4**

| Amount of W loading / % | Isobutene conversion / % | Selectivity / % | | | |
|---|---|---|---|---|---|
| | | C₈ | | C₁₂ | C_{16 & +} |
| | | 2,4,4-Trimethyl pentene | Others | | |
| 0.5 | 52.1 | 90.8 | 0 | 9.2 | 0 |
| 0.75 | 52.4 | 88.1 | 0.5 | 11.4 | 0 |
| 1.0 | 52.4 | 89.8 | 0.6 | 9.2 | 0.4 |

### Example 5

Effects of Si/Al ratio on the performance of Co / H-ZSM-5 (metal loading of 0.75 %) zeolite catalyst prepared by ion-exchange technique were investigated as shown in Table 5.

The conditions for the pre-treatment and the dimerization were the same as in Example 1.

**Table 5**

| Zeolite catalyst / (Si/Al ratio) | Isobutene conversion / % | Selectivity / % | | | |
|---|---|---|---|---|---|
| | | C₈ | | C₁₂ | C_{16 & +} |
| | | 2,4,4-Trimethyl pentene | Others | | |
| Co / H ZSM-5 (45) | 54.4 | 91.2 | 0.5 | 7.9 | 0.4 |
| H-ZSM-5 (45) | 47.0 | 88.9 | 0.6 | 8.4 | 2.1 |
| Co / H ZSM-5 (27.5) | 83.6 | 53.6 | 0.3 | 45.5 | 0.6 |
| H-ZSM-5 (27.5) | 72.8 | 39.4 | 0.2 | 36.8 | 23.6 |

### Example 6

Effects of feed composition on the performance of Co / H-ZSM-5 (Si/Al ratio of 45, metal loading of 0.75 %) zeolite catalyst prepared by ion-exchange technique were investigated as shown in Table 6.

The conditions for the pre-treatment and the dimerization were the same as in Example 1.

**Table 6**

| Isobutene/Isobutane / Weight ratio | Isobutene conversion /% | Selectivity / % | | | |
|---|---|---|---|---|---|
| | | C8 | | C12 | Others (C12+) |
| | | 2,4,4-Trimethyl pentene | Others | | |
| 40 : 60 | 53.1 | 92.9 | 0.5 | 6.6 | 0 |
| 50: 50 | 54.4 | 91.2 | 0.5 | 7.9 | 0.4 |
| 60 : 40 | 81.5 | 62.4 | 3.3 | 30.7 | 3.6 |

### Example 7

Effects of reaction temperature on the performance of Co / H-ZSM-5 (Si/Al ratio of 45, metal loading of 0.75 %) zeolite catalyst prepared by ion-exchange technique were investigated.

The conditions for the pre-treatment and the dimerization were the same as in Example 1, except for the dimerization temperature which was varied as shown in Table 7.

**Table 7:**

| Reaction temperature / oC | Isobutene conversion /% | Selectivity / % | | | |
|---|---|---|---|---|---|
| | | C8 | | C12 | Others (C12+) |
| | | 2,4,4-Trimethyl pentene | Others | | |
| 50 | 54.4 | 91.2 | 0.5 | 7.9 | 0.4 |
| 60 | 65 | 87.2 | 0.5 | 12.1 | 0.2 |
| 70 | 75.7 | 81.9 | 0.7 | 16.0 | 1.4 |

### Example 8

Results of isobutene dimerization over cobalt containing H-ZSM-5 (Si/Al ratio of 45, metal loading of about 0.75%) catalysts prepared by various techniques are shown in Table 8.

The conditions for the pre-treatment and the dimerization were the same as in Example 1.

**Table 8**

| Catalyst preparation technique | Isobutene conversion /% | Selectivity/% | | | |
|---|---|---|---|---|---|
| | | C₈ | | C₁₂ | C_{16 & +} |
| | | 2,4,4-Trimethyl pentene | Others | | |
| Ion-exchange | 54.4 | 91.2 | 0.5 | 7.9 | 0.4 |
| Wetness impregnation | 43.1 | 92.2 | 0.3 | 6.6 | 0.9 |
| Sonification | 47 | 92.6 | 0.4 | 7 | 0 |
| Precipitation | 51.0 | 90.5 | 0.5 | 5.6 | 3.4 |

## Claims

1. Process for the dimerization of olefins or a mixture of olefins and paraffins, wherein said olefins and paraffins have between 2 to 10 carbon atoms, comprising contacting a feedstream comprising said olefin or mixture of olefins and paraffins with a catalyst composition comprising a medium pore aluminosilicate zeolite and up to 5 wt-% of one or more elements selected from Groups 6 and 9 of the Periodic Table.

2. The process according to claim 1, wherein said one or more elements are selected from the group consisting of molybdenum (Mo), tungsten (W), cobalt (Co) and rhodium (Rh).

3. The process according to claim 1 or 2, wherein the medium pore aluminosilicate zeolite is a 10-ring zeolite having pores formed by a ring consisting of 10 SiO₄ tetrahedra.

4. The process according to any one of claims 1-3, wherein the medium pore aluminosilicate zeolite has a pore size of between more than 5 Å but less than 6 Å and preferably between 5.2 and 5.8.

5. The process according to any one of claims 1-4, wherein the zeolite is of the ZSM-5 type.

6. The process according to any one of claims 1-5, wherein the silicon to aluminium (Si:Al) molar ratio of the zeolite is 10-100, preferably 30-60 and most preferably 40-50.

7. The process according to any one of claims 1-6, wherein the process is performed under conditions comprising a temperature of 40-80 °C and a pressure of 1-2.5 MPa.

8. The process according to any one of claims 1-7, wherein the catalyst composition before contacting with the feedstream is pre-treated with an inert gas comprising water vapour or alcohol vapour at a temperature between 30 °C and the boiling temperature of water or the alcohol, preferably 30-90 °C, more preferably 40-70 °C.

9. The process according to claim 8 wherein the alcohol is selected from the group consisting of 1-propanol, isopropanol, isobutanol and tertiary butanol or a mixture thereof.

10. The process according to claim 8 or 9, wherein the inert gas is selected from the group consisting of nitrogen, helium and argon or a mixture thereof.

11. The process according to any one of claims 1-10, wherein the molar ratio of the olefins to paraffins in the feedstream is 1:0.1-10, preferably 1:0.2-5, and most preferably 1:0.5-2.

12. The process according to claim 11, wherein the feedstream comprises isobutene and isobutane.

13. A catalyst composition comprising a medium pore aluminosilicate zeolite and up to 5 wt-% of one or more elements selected from Groups 6 and 9 of the Periodic Table, wherein the catalyst composition is pre-treated with an inert gas comprising water vapour or alcohol vapour at a temperature of 30 °C to the boiling temperature of water/alcohol, preferably 40-70 °C.
